# EUROPÄISCHE PATENTSCHRIFT

(11) **EP 3 590 560 B1**
(45) Veröffentlichungstag und Bekanntmachung des Hinweises auf die Patenterteilung: **03.03.2021**
(21) Anmeldenummer: 19178887.6
(22) Anmeldetag: 09.05.2016
(51) Int. Cl.: A61M 1/16, B01D 63/02, B01D 63/04

(54) **TRAGBARE GASAUSTAUSCHVORRICHTUNG**
PORTABLE GAS EXCHANGE APPARATUS
DISPOSITIF PORTATIF D'ÉCHANGE GAZEUX

(30) Priorität: 07.05.2015 EP 15001370
(43) Veröffentlichungstag der Anmeldung: 08.01.2020
(62) Teilanmeldung aus: 16721082.2
(73) Patentinhaber: novalung GmbH, 74076 Heilbronn (DE)
(72) Erfinder: Matheis, Georg, 74076 Heilbronn (DE); Thumm, Tatjana, 72644 Oberboihingen (DE); Hildebrand, Michael, 72116 Mössingen (DE); Panagias, Nektarios, 72762 Reutlingen (DE); Stingel, Dirk, 72469 Tieringen (DE); Beuter, Reinhold, 72414 Rangendingen (DE)
(74) Vertreter: Graf von Stosch Patentanwaltsgesellschaft mbH

(56) Entgegenhaltungen:
- WO-A1-2013/041950
- WO-A1-2014/012536
- WO-A2-2014/177944
- US-A- 5 308 314
- US-A1- 2002 161 349
- US-A1- 2009 081 079
- US-A1- 2010 101 657

## Beschreibung

### Hintergrund der Erfindung

Die Erfindung betrifft eine Gasaustauschvorrichtung zur Behandlung einer insbesondere biologischen Flüssigkeit.

Bei einer Gasaustauschvorrichtung kann es sich um eine Begasungs- bzw. Entgasungsvorrichtung handeln, bei der ein oder mehrere Gase von einem Medium in ein anderes Medium übertreten können, bzw. um eine Vorrichtung, die den Austausch eines oder mehrerer Gase zwischen zwei Medien ermöglicht. Solche Vorrichtungen finden Anwendung in der Chemie, der Biotechnologie und der Medizin. Ein wichtiger Einsatzzweck in der Medizin ist die Anreicherung einer biologischen Flüssigkeit, insbesondere von Blut, mit Sauerstoff und/oder die Entfernung, also Abreicherung, von Kohlenstoffdioxid aus der Flüssigkeit, speziell Blut. Solche Maßnahmen werden bspw. bei der Behandlung von verschiedenen Lungenerkrankungen notwendig. Solche Maßnahmen können weiterhin z.B. auch bei akutem Lungenversagen, sowie zum Ersatz der Lunge, während deren Umgehung mit einem extrakorporalen Kreislauf, mit unterschiedlichem Ausmaß bei der mechanischen Herzunterstützung und zur Ermöglichung am stillgestellten Herzen zu operieren, notwendig sein.

Die derzeit einzige langfristig effektive Therapieoption für Patienten mit endgradiger funktioneller Lungenerkrankung stellt die Lungentransplantation dar. Eine andere medizinische Lösung, um dauerhaft die Funktion der Lunge zu ersetzen, existiert hingegen nicht. Bei Patienten, die unter chronischen Lungenerkrankungen leiden und nicht oder nicht unmittelbar für eine Lungentransplantation in Betracht kommen, besteht daher ein Bedürfnis nach künstlichen Lungenersatzverfahren.

Um ein derartiges Lungenersatzverfahren zu ermöglichen, sind so genannte Blutgastauscher aus dem Stand der Technik bekannt.

Ein Blutgastauscher, auch als Oxygenator oder künstliche Lunge bezeichnet, wird entweder zur vollständigen, kurzzeitigen Übernahme der Lungenfunktion während einer Operation am offenen Herzen oder als vollständige oder teilweise, langfristige Unterstützung der Lunge auf der Intensivstation eingesetzt. Die Hauptfunktion eines Blutgastauschers besteht in der Abgabe von Sauerstoff an das Blut (Oxygenierung) und in der Aufnahme von Kohlenstoffdioxid aus dem Blut (Decarboxylierung). Der Gasaustausch findet beispielsweise mittels Hohlfasermembranen statt, die im extrakorporalen Gasaustauscher von Blut umflossen werden, während gleichzeitig durch das Innere der Faser sauerstoffreiches bzw. kohlenstoffdioxidarmes Gas geleitet wird. Durch den Konzentrationsunterschied können Sauerstoff bzw. Kohlenstoffdioxid in jeweils entgegengesetzter Richtung durch eine teildurchlässige Membran - typischerweise eine gaspermeable Membran - diffundieren. Dazu verwendete so genannte Membranplatten lassen sich kommerziell beziehen.

Neben einer Anwendung zur Oxygenierung und Decarboxylierung ist es in einigen Therapieansätzen bereits ausreichend, lediglich eine Decarboxylierung vorzunehmen. Dabei handelt es sich um eine so genannte extrakorporale Reduktion von CO2 (ECCO₂R). Dazu wird über einen venösen Zugang des Patienten kontinuierlich Blut entnommen, extrakorporal durch einen Blutgastauscher gepumpt, dort von CO2 gereinigt und dem Patienten venös wieder zugeführt.

Als Pumpen werden bei den gängigen ECCO₂R - Anwendungen so wie auch bei der so genannten Extrakorporalen Membranoxygenierung (ECMO) häufig Systeme und insbesondere Pumpen verwendet, die auch bei der Herzchirurgie als Teil einer Herz-Lungen-Maschine im Rahmen herzchirurgischer Eingriffe am offenen Herzen bei Kreislaufstillstand vorübergehend die Pumpfunktion des Herzens übernehmen können. Diese Pumpsysteme sind auf die notwendigen Volumenströme von bis zu 6 l/min für die ausreichende Oxygenierung des Blutes bei Kreislaufstillstand ausgelegt und dimensioniert.

Für den Gasaustausch in derartigen Blutgastauschern wird üblicherweise Sauerstoff verwendet, der in jedem Krankenhaus entweder direkt über einen Wandanschluss oder Sauerstoffflaschen zur Verfügung steht. Der Sauerstoff wird dann über einen regelbaren Gasblender dem Blutgastauscher zugeführt.

Pumpsysteme für ECCO₂R - Anwendungen werden grundsätzlich am stationären Stromnetz betrieben. Im Falle eines Notfalls, wie beispielsweise einem Stromausfall, kann eine Notstromversorgung mittels Batteriebetrieb aufrechterhalten werden. Da Krankeneinrichtungen in der Regel über Notstromgeneratoren verfügen, ist ein Batteriebetrieb zeitlich jedoch limitiert und bei den bekannten Gasaustauschvorrichtungen in der Regel nicht zum Dauerbetrieb oder zum mobilen Betrieb ausgelegt. Ein bekanntes Blutgastauschsystem, das zwar über eine mobile Stromversorgung verfügt, erfordert jedoch das Mitführen einer beispielsweise rollbaren Trägereinrichtung, um die Vielzahl verschiedener, zum Teil sperriger, Komponenten des Blutgastauschers mitzuführen.

Auf Grund ihrer Größe, ihres Gewichts und auch wegen der Anordnung von Anschlüssen beispielsweise für blutführende Schläuche, beschränkt sich der Einsatz der bekannten Gasaustauschvorrichtungen daher im Wesentlichen auf stationäre Anwendungen, bei denen der Patient - wach oder sediert - im Bett liegt. Patienten mit chronischen Lungenerkrankungen, die auf eine dieser bekannten Gasaustauschvorrichtungen angewiesen sind, sind somit stark in ihrer Mobilität eingeschränkt. Dies vermindert nicht nur die Lebensqualität der Patienten erheblich. Auch moderne Therapieansätze, die eine Mobilisierung der Patienten anstreben, sind nicht durchführbar.

Die bekannten Gasaustauschvorrichtungen sind dabei insbesondere auch zu voluminös und zu schwer, um von einem Patienten, insbesondere über einen längeren Zeitraum, am Körper getragen werden zu können. Selbst Therapieansätze im Falle chronischer Lungenkrankheiten, die lediglich eine Entlastung und nicht den Vollersatz der Lunge bezwecken, erfordern daher eine stationäre Anwendung.

Tragbare Gasaustauschvorrichtungen sind z.B. aus US 2009/0081079 (A1) und WO 2014/177944 (A2) bekannt.

### Kurze Beschreibung der Erfindung

Der vorliegenden Erfindung liegt die Aufgabe zugrunde, ein verbessertes Pumpsystem bzw. eine verbesserte Gasaustauschvorrichtung bereitzustellen, um wenigstens einen der Nachteile der bekannten Systeme zu lösen und eine mobile Anwendung zu ermöglichen. Es ist insbesondere eine Aufgabe der vorliegenden Erfindung, einen Platzbedarf und ein Gewicht eines Pumpsystems in einer Gasaustauschvorrichtung zu reduzieren. Es ist zudem eine Aufgabe eine Gasaustauschvorrichtung bereitzustellen, die von einem Patienten auch über einen längeren Zeitraum hinweg, vorzugsweise direkt, an dem Patientenkörper transportiert werden kann.

Diese Aufgabe wird gelöst durch eine Gasaustauschvorrichtung gemäß Anspruch 1. Vorteilhafte Weiterbildungen der Erfindung sind Gegenstand der abhängigen Ansprüche.

Ein Gasaustausch kann dem konvektiven und diffusiven Stoffaustausch unterliegen. Vorzugsweise ist der Gasaustausch diffusiv und wird über die Differenz der Gaskonzentration auf beiden Seiten einer Membran bestimmt. Für die vorliegende Erfindung bedeutet dies, dass sich in einem System aus verschiedenen gekoppelten Fluiden, beispielsweise eine Flüssigkeit und ein Gas, ein Gleichgewicht der jeweiligen - gasförmigen - Komponenten der Fluide einstellen wird. Wird nun eine Flüssigkeit mit einem erhöhten Anteil eines Gases von einem Gas umströmt, das einen sehr geringen Anteil dieses Gases enthält, so wird der Gasanteil dieses spezifischen Gases aus der Flüssigkeit in das umströmende Gas so lange übertreten, bis ein Konzentrationsausgleich stattgefunden hat. Wird der mit dem Gas angereicherte Gasstrom durch ein von dem Gas abgereichertes Gasgemisch ersetzt, bleibt das Konzentrationsverhältnis zwischen Flüssigkeit und umströmendem Gasgemisch unverändert hoch und es kommt zu einer kontinuierlichen Abgabe von Gas aus der Flüssigkeit.

Gemäß der vorliegenden Erfindung wird eine tragbare Gasaustauschvorrichtung zum Gasaustausch wenigstens eines Gases aus einer Flüssigkeit mit einem Fluid bereitgestellt. Die Gasaustauschvorrichtung weist einen ersten Gasaustauschabschnitt, zum Durchfluss eines Fluids, und einen zweiten Gasaustauschabschnitt zum Durchfluss einer mit einem ersten Gas angereicherten Flüssigkeit, auf. Die Gasaustauschvorrichtung weist zudem ein Pumpsystem auf, das zumindest zum Transport des Fluids mit einer geringen Konzentration eines ersten Gases in dem ersten Gasaustauschabschnitt ausgebildet ist. In dem ersten Gasaustauschabschnitt ist ein erster Fluidkreislauf ausgebildet sein, durch den das Fluid strömt. In einer Mischkammer der Gasaustauschvorrichtung grenzen der erste Gasaustauschabschnitt und der zweite Gasaustauschabschnitt aneinander und sind durch eine zumindest von dem ersten Gas passierbar ausgebildete Wandung getrennt. Zudem weist die Gasaustauschvorrichtung ein Gehäuse zur Aufnahme wenigstens des Pumpsystems und der Mischkammer auf. Die Aufnahme erfolgt vorzugweise gemeinsam in demselben Gehäuse.

Weiterhin weist eine tragbare Gasaustauschvorrichtung nach der vorliegenden Erfindung eine Heizvorrichtung zum Aufwärmen des Fluids auf. Die Heizvorrichtung wird dadurch bereitgestellt, dass ein Antrieb der ersten Pumpe (20) derart in dem Gehäuse (2) der Gasaustauschvorrichtung (1) positionierbar ist, dass eine Abwärme des Antriebs der ersten Pumpe (20) einen einlassseitigen Abschnitt des ersten Fluidkreislaufs erwärmt, wobei der einlassseitige Abschnitt in stromaufwärtiger Richtung des ersten Fluidkreislaufs vor dem ersten Gasaustauschabschnitt ausgebildet ist.

Vorzugsweise weist das Gehäuse ein maximales Innenraumvolumen von bevorzugt 9500 cm³ auf, bei einer bevorzugten maximalen durchschnittlichen Tiefe von 15 cm und bei einem bevorzugten durchschnittlichen summarischen Wert für Höhe und Breite von zusammengenommen maximal 65 cm. Die durchschnittliche Höhe oder die durchschnittliche Breite betragen hierbei bevorzugt maximal 40 cm. Die Tiefe beträgt dabei bevorzugt 1/4 bis 1/8 des summarischen Wertes der durchschnittlichen Höhe und der durchschnittlichen Breite zusammengenommen, so dass das Gehäuse einen gedrungenen bis flächigen Habitus verwirklicht. In diesem kompakten Gehäuse werden im Wesentlichen alle funktionalen Einheiten der Gasaustauschvorrichtung integriert, wodurch ermöglicht wird, dass ein Patient die Gasaustauschvorrichtung komfortabel am Körper tragen kann.

Unter einer "geringen Konzentration" ist dabei hier zu verstehen, dass das in dem ersten Gasaustauschabschnitt vorgesehene Fluid mit einer geringeren, vorzugsweise deutlich geringeren, Konzentration des Gases vorgesehen ist, als die Flüssigkeit in dem zweiten Gasaustauschabschnitt.

In einem derartigen Pumpsystem können der erste und ein zweiter Fluidkreislauf zumindest teilweise aneinander angrenzen und durch eine selektiv-permeable Wandung getrennt sein. Der zweite Fluidkreislauf ist dabei vorzugsweise als ein Teil des zweiten Gasaustauschabschnitts ausgebildet. Das Pumpsystem weist wenigstens eine Pumpeinrichtung auf, die zum Transport eines ersten Fluids mit einer geringen Konzentration eines ersten Gases in dem ersten Gasaustauschabschnitt bzw. dem ersten Fluidkreislauf ausgebildet ist. Zudem kann das Pumpsystem eine mobile Stromversorgung aufweisen. Die Stromversorgung kann somit einen autarken betrieb des Pumpsystems ermöglichen.

Eine Trennung zwischen zwei Fluiden, die den gleichen Aggregatszustand oder unterschiedliche Aggregatszustände besitzen, wird im Sinne der Erfindung auch als eine "selektiv permeable Wandung" bezeichnet. Diese Trennung erlaubt vorzugsweise ein Durchtreten von vorbestimmten Komponenten, insbesondere spezifischer Gasmoleküle. Dabei kann es sich insbesondere um eine Wandung handeln, die durchlässig ist für Blutgase, wie beispielsweise Kohlenstoffdioxid oder Sauerstoff. Es versteht sich, dass die Wandungen abhängig von dem spezifischen Einsatzgebiet auch permeabel für zusätzliche oder andere Gase oder Flüssigkeiten bzw. Komponenten daraus sein kann.

Als ein Fluidkreislauf wird im Sinne der Erfindung eine Anordnung verstanden, die das Durchtreten eines Fluids, also einer Flüssigkeit oder eines Gases bzw. Gasgemisches, von einem Zufluss oder Einlass zu einem Abfluss oder Auslass erlaubt. Dabei muss es sich jedoch nicht um einen geschlossenen Kreislauf handeln. Vielmehr können im Sinne der Erfindung ein Einlass und ein Auslass örtlich voneinander getrennt angeordnet sein, wobei im Sinne der Erfindung dennoch von einem Kreislauf gesprochen werden kann.

Eine mobile Stromversorgung im Sinne der Erfindung kann beispielsweise eine Batterie aufweisen, vorzugsweise einen wieder aufladbaren Akkumulator, beispielsweise einen Lithium-Ionen Akku. Vorteilhafterweise kann die mobile Stromversorgung wenigstens zwei separate und separat entfernbare Batterien, vorzugsweise wieder aufladbare Akkumulatoren, aufweisen. In diesem Fall kann einer der Akkumulatoren außerhalb der Vorrichtung aufgeladen werden, während der entsprechend andere Akkumulator die Energieversorgung des Pumpsystems übernimmt. Dabei kann die Stromversorgung dazu ausgelegt sein, dass ein Austausch der Batterien möglich, während das System funktionsfähig bleibt ("Hot-Swapping"). Die Batterie bzw. die Batterien können auch zur Stromversorgung einer Steuereinheit des Pumpsystems oder zur Stromversorgung der gesamten erfindungsgemäßen Gasaustauschvorrichtung bzw. deren Steuereinheit dienen.

Gängige Systeme, insbesondere auch zur extrakorporalen Reduktion von CO₂, sind stationäre Systeme, die grundsätzlich nicht für einen mobilen Einsatz geeignet sind, und insbesondere nicht für einen Einsatz, während dessen sie von einem Patienten mitgeführt oder sogar getragen werden. Entsprechend sind diese bekannten Systeme weder mit einer mobilen Stromversorgung vorgesehen, noch sind Komponenten enthalten, die dauerhaft ohne einen direkten Netzanschluss betrieben werden können. Erfindungsgemäß wird durch das Vorsehen einer mobilen Stromversorgung ermöglicht, dass ein Gasaustausch zwischen einer Flüssigkeit, beispielsweise einer biologischen Flüssigkeit, wie zum Beispiel Blut, und einem Fluid, wie beispielsweise einem Gas, standortunabhängig erfolgen kann. Das Fluid kann dabei beispielsweise ein Gas oder Gasgemisch sein, wie Sauerstoff, Umgebungsluft, mit Sauerstoff angereicherte Umgebungsluft oder von Kohlenstoffdioxid oder von anderen Bestandteilen abgereicherte Umgebungsluft, und andere. Ein Pumpbetrieb und somit ein Gasaustausch kann daher erfindungsgemäß dauerhaft auch dann erfolgen, wenn ein Patient nicht mit einer stationären, stromnetz-gekoppelten Stromversorgung verbunden ist.

Bevorzugte Ausführungsformen der erfindungsgemäßen Vorrichtung dienen ausschließlich zur extrakorporalen Reduzierung des Kohlenstoffgehalts im Blut. Die hier dargestellten Weiterbildungen und Vorteile gelten jedoch im selben Maße auch für Systeme, die alternativ oder zusätzlich zu einer Gasabreicherung von der Flüssigkeit, wie beispielsweise der oben genannte Decarboxylierung, eine Gasanreicherung in der Flüssigkeit bewirken können, wie beispielsweise eine Oxygenierung von Blut. Auch Systeme zur Oxygenierung oder Systeme zur Anreicherung und/oder Abreicherung mit anderen Stoffen, Gasen oder Molekülen sind insofern grundsätzlich von dem erfindungsgemäßen Gedanken eine tragbare Gasaustauschvorrichtung bereitzustellen, umfasst.

Insbesondere das Vorsehen eines Gehäuses, in dem die Pumpeinrichtung und die Mischkammer, vorzugsweise gemeinsam mit den entsprechenden Zu- und Ableitungen, aufnehmbar sind, erlaubt eine derartige kompakte Anordnung der notwendigen Komponenten zum extrakorporalen Gasaustausch, dass eine Vorrichtung geschaffen werden kann, die von einem Patienten tragbar ist, die also nicht mittels medizinischer Hilfsgerätschaften oder von Begleitpersonen getragen werden muss. Die Gasaustauschvorrichtung in dem Gehäuse kann erfindungsgemäß insbesondere auch direkt am Körper eines Nutzers getragen werden. Dazu kann die Gasaustauschvorrichtung und insbesondere das Gehäuse auch geeignete Haltemittel zur Befestigung an einem Körper, an Kleidung oder an Taschen des Patienten aufweisen.

In einigen Weiterbildungen der Erfindung weist der erste Gasaustauschabschnitt, insbesondere der erste Fluidkreislauf, einen Partikelfilter auf. Der Partikelfilter ist vorzugsweise einlassseitig, insbesondere in, an, auf oder vor einem Ansaug- oder Anschlussabschnitt des ersten Fluidkreislaufs ausgebildet. Der Partikelfilter ermöglicht dabei ein Filtern des Fluids. Das Fluid kann dabei insbesondere im Wesentlichen Umgebungsluft, Sauerstoff, oder eine Mischung aus Umgebungsluft und Sauerstoff aufweisen. Es versteht sich, dass verschiedenste Gase und Gasgemische auf dieselbe Weise mit der erfindungsgemäßen Gasaustauschvorrichtung verwendbar sind. Durch das Vorsehen eines Partikelfilters in dem ersten Fluidkreislauf kann vermieden werden, dass Fremdkörper, wie beispielsweise Staub, Feinstaub oder andere Schwebeteilchen, in einen Bereich der Gasaustauschvorrichtung gelangen, in dem ein Gasaustausch zwischen dem ersten Fluidkreislauf und dem zweiten Fluidkreislauf erfolgt. So kann ein Verstopfen des eigentlichen Gasaustauschbereichs, insbesondere in der Mischkammer, zwischen den Fluidkreisläufen reduziert oder verhindert werden. Dies ist insbesondere in einem Fall vorteilhaft, in dem das erste Fluid Umgebungsluft aufweist oder zumindest zu einem wesentlichen Teil aus Umgebungsluft besteht, die auf autarke Weise von der Gasaustauschvorrichtung aus der Umgebung abgepumpt wird.

Als "einlassseitig" wird dabei ein Abschnitt des betreffenden, hier des ersten, Fluidkreislaufs bezeichnet, der auf einer Seite des Einlasses, also in stromaufwärtiger Richtung des Fluidkreislaufes vor einem Gasaustauscher, ausgebildet ist. Dies umfasst den Einlass an sich, jedoch auch beispielsweise einen Leitungsabschnitt, der das Fluid von dem Einlass zu dem Gasaustauscher leitet. Der Einlass kann dabei als ein Ansaugabschnitt oder ein Fluidanschluss, beispielsweise ein Gasanschluss, ausgebildet sein. Mittels eines Ansauganschlusses kann beispielsweise Luft aus der Umgebung des Ansaugabschnitts in das Pumpsystem gesogen werden. Ein Fluidanschluss kann den direkten Anschluss zur Versorgung mit einem mitgeführten Fluid zusätzlich, separat oder optional, erlauben. Auf diese Weise kann die angesaugte Luft beispielsweise mit einer gewünschten Komponente, beispielsweise Sauerstoff, angereichert werden.

Analog bezeichnet "auslassseitig" einen Abschnitt des entsprechenden Fluidkreislaufs, der stromabwärts von einem Gasaustauscher ausgebildet ist. Dies umfasst einen Auslass oder einen Auslassanschluss ebenso wie einen zu dem Auslass führenden Leitungsabschnitt. Das durch den Auslassanschluss geleitete Gas kann zumindest teilweise wieder dem Fluidkreislauf an dem Einlassabschnitt zugeführt werden. Dies kann insbesondere bei kalten Umgebungstemperaturen eine Energie zum Erwärmen der zugeführten oder angesaugten Luft reduzieren.

Unter "Umgebungsluft" wird dabei im Sinne der Erfindung ein typisches Luftgemisch aus Stickstoff, Sauerstoff, Kohlenstoffdioxid und weiterer Komponenten bezeichnet, das üblicherweise auch als Atemluft qualifiziert ist. Insbesondere bezeichnet der Begriff "Umgebungsluft" erfindungsgemäß ein derartiges Gasgemisch, das einen Kohlenstoffdioxid-Gehalt von typischerweise weniger als 0,5-1 Vol-%, insbesondere von ca. 0,04 Vol-% aufweist.

Die erfindungsgemäße Gasaustauschvorrichtung, insbesondere das Pumpsystem, kann eine erste Pumpe (20) zum Pumpen des Fluids, vorzugsweise eines Gases, z.B. Umgebungsluft, aufweisen.

Die erfindungsgemäße Gasaustauschvorrichtung, insbesondere das Pumpsystem, kann eine zweite Pumpe zum Pumpen einer Flüssigkeit, vorzugsweise einer biologischen Flüssigkeit, durch den zweiten Gasaustauschabschnitt, vorzugsweise einen zweiten Fluidkreislauf, aufweisen. Auf diese Weise kann ermöglicht werden, dass ein verbesserter Transport der Flüssigkeit, die insbesondere eine biologische Flüssigkeit wie Blut sein kann, durch das Pumpsystem und daran angeschlossene Komponenten, erfolgen kann. Auf diese Weise kann, sofern es sich bei der Flüssigkeit um Blut eines Patienten handelt, eine Pumpleistung des Herzens des Patienten unterstützt, bzw. kann das Herz eines Patienten zumindest entlastet werden. Zudem kann ein Fluss durch den zweiten Gasaustauschabschnitt gleichmäßiger und unabhängig von einem Herzschlag erfolgen.

In einigen Weiterbildungen der Erfindung kann die erste und/oder die zweite Pumpe des Pumpsystems ein Steuermittel zum Steuern der Flussrate des jeweiligen von der Pumpe transportierten Fluids aufweisen. Dadurch kann durch entsprechendes Ansteuern der ersten und/oder der zweiten Pumpe eine Abreicherungsrate des ersten Gases eingestellt werden. Konkret kann dies bedeuten, dass, im Falle eines erhöhten Gasanteils in der Flüssigkeit, beispielsweise eine erhöhte Kohlenstoffdioxidanreicherung in dem Blut, eine Flussrate der ersten Pumpe erhöht wird. Der Konzentrationsunterschied des ersten Gases in der Flüssigkeit und in dem angesaugten Gas kann dadurch erhöht werden und es kann im vorliegenden Beispiel eine verbesserte Kohlenstoffdioxidabreicherung stattfinden. Durch Erhöhen der Pumpleistung der zweiten Pumpe kann gleichsam auf eine erhöhte Kohlenstoffdioxidanreicherung in der Flüssigkeit reagiert werden. Bei erhöhter Pumpleistung kann ein erhöhtes Flüssigkeitsvolumen durch den Gasaustauscher transportiert werden. Dies kann beispielsweise dann notwendig werden, wenn sich der Patient körperlich betätigt.

Es versteht sich, dass die erste und die zweite Pumpe unabhängig voneinander oder auch gemeinsam, insbesondere gleichzeitig, angesteuert werden können. Dies kann auch ermöglichen, dass aus jedem Flüssigkeitsflussvolumen, insbesondere Blutflussvolumen, das Verhältnis von Gas zu Blutfluss durch den Gasaustauscher optimiert wird. Dies kann eine Invasivität durch eine externe Gasaustauschvorrichtung reduzieren, beispielsweise dadurch, dass ein möglichst kleinlumiger Gefäßzugangs zu einem Patienten verwendet werden kann. Dies kann beispielsweise durch einen so genannten Master-Slave Betrieb des Pumpsystems bzw. der Fluidkreisläufe mit diversen Sensoren realisiert werden. Demgemäß kann eine, beispielsweise maximale und/oder minimale und/oder optimale, Flussrate einer der Pumpen abhängig von einer Einstellung oder einer, beispielsweise maximalen und/oder minimalen und/oder eingestellten, Förderrate der entsprechend anderen Pumpe einstellbar sein. Es versteht sich, dass die entsprechenden korrespondierenden Pumpleistungen der ersten und der zweiten Pumpe, auch beispielsweise in Abhängigkeit eines Lumen eines Gefäßzuganges, in einer Speichereinheit einer Steuereinheit hinterlegt sein können. Auf diese Weise kann beispielsweise eine teil- oder vollautomatische Einstellung der Pumpenparameter erfolgen. Dies kann den Bedienkomfort der Vorrichtung erhöhen. Zudem kann auf diese Weise eine verbesserte, insbesondere eine optimale, Gasaustauschleistung bei geringem Blutfluss, insbesondere bei geringstmöglichem Blutfluss, erreicht werden.

Die Flussrate der ersten Pumpe kann dabei im Bereich zwischen 1-12 l/min, vorzugsweise zwischen ca. 1 - 8 l/min einstellbar sein. Dies kann insbesondere dann der Fall sein, wenn die erste Pumpe zum Pumpen eines Gases vorgesehen und ausgebildet ist. In alternativen Ausführungsformen ist es denkbar, dass es sich bei dem Fluid in dem ersten Fluidkreislauf um eine Flüssigkeit handelt, beispielsweise eine mit Gas an- oder abgereicherte Flüssigkeit. Ein Pumpvolumen kann dann entsprechend in anderen adäquaten Bereichen einstellbar sein.

Die erste Pumpe ist insbesondere eine Pumpe mit einem Leistungsgewicht kleiner 30 kg/KW bei einem Pumpengewicht von höchstens 0,5 kg, vorzugsweise höchstens 0,3 kg. Durch die Verwendung einer Pumpe mit einem Leistungsgewicht kleiner 30 kg/KW bei einem maximalen Pumpengewicht von 0,5 kg, vorzugsweise maximal 0,3 kg kann eine kompakte und leichte Bauweise der Gasaustauschvorrichtung erzielt werden, die es ermöglicht, dass die Gasaustauschvorrichtung von einer Person ohne Kraftaufwand getragen werden kann.

In einigen Ausführungsformen der vorliegenden Erfindung ist die Flussrate der zweiten Pumpe geringer als 4 l/min und vorzugsweise geringer als 2 l/min. Die Flussrate der zweiten Pumpe ist dabei vorzugsweise steuerbar und/oder regelbar. Die Flussrate der zweiten Pumpe liegt dabei vorzugsweise 0,2 bis 2,0 l/min. Vorteilhafterweise ist die Flussrate der zweiten Pumpe zwischen ca. 0,2-1,5 l/min, vorzugsweise zwischen ca. 0,3 - 1 l/min einstellbar. Vorzugsweise beträgt dabei auch die maximal erreichbare Flussrate der zweiten Pumpe weniger als 4 l/min, bevorzugt weniger als 2 l/min, und insbesondere höchstens 1,5 l/min. Eine derartige Pumpe, die wesentlich geringere maximale Flussraten aufweist als die bekannten für diesen Einsatzzweck vorgesehenen Pumpen, kann eine erhebliche Reduzierung der Baugröße, des Baugewichts oder auch des Energieverbrauchs erlauben. Dies kann durch daraus resultierende längere Pumpleistungen eine Mobilität des Pumpsystems erhöhen. Das Vorsehen von Pumpen mit geringerer Pumpleistung gemäß der vorliegenden Erfindung kann zudem auch eine Reduktion der Größe der verwendeten Pumpen mit sich bringen. Dies kann es erlauben, das Gewicht der Pumpeinrichtung und somit des gesamten Systems reduzieren. Dies kann einen Transport des gesamten Pumpsystems erleichtern und insbesondere eine Tragbarkeit direkt am Körper eines Patienten ermöglichen.

Die zweite Pumpe ist insbesondere eine Pumpe mit einem Leistungsgewicht kleiner 25 kg/KW bei einem Pumpengewicht von höchstens 0,7 kg, vorzugsweise höchstens 0,5 kg. Durch die Verwendung einer Pumpe mit einem Leistungsgewicht kleiner 25 kg/KW und einem maximalen Pumpengewicht von 0,7 kg, vorzugsweise maximal 0,5 kg kann eine kompakte und leichte Bauweise der Gasaustauschvorrichtung erzielt werden, die es ermöglicht, dass die Gasaustauschvorrichtung von einer Person ohne Kraftaufwand getragen werden kann.

Die Reduktion der Pumpengröße kann es zudem ermöglichen, die Pumpeinrichtung leichter gemeinsam mit einer Gasaustauscheinrichtung in einem gemeinsamen Gehäuse unterzubringen. Dies kann eine Tragbarkeit des Systems weiter verbessern und einen Komfort für den jeweiligen Träger erhöhen.

Das Gehäuse kann dabei beispielsweise einen Gehäuseeinsatz aufweisen, der mit Aufnahmeausnehmungen für verschiedene Komponenten der Gasaustauschvorrichtung ausgebildet ist. Insbesondere kann der Gehäuseeinsatz einstückig sein. Das Gehäuse bzw. der Gehäuseeinsatz kann beispielsweise einen Schaumstoff oder ein Formgussmaterial aufweisen. Dabei kann das Gehäuse bzw. der Gehäuseeinsatz derart vorgesehen sein, beispielsweise durch Vorsehen von entsprechenden Ausformungen in dem Gehäuse bzw. dem Gehäuseeinsatz, dass eine formschlüssige Aufnahme der Komponenten der Gasaustauschvorrichtung ermöglicht ist. In einigen Ausführungsformen kann das Gehäuse einstückig mit dem Gehäuseeinsatz ausgebildet sein.

Das Gehäuse bzw. das Material des Gehäuseeinsatzes kann ein Schall- und/oder Wärme-isolierendes Material aufweisen. Zudem kann das Material des Gehäuseeinsatzes ein stoßfestes und/oder wasserabweisendes Material aufweisen.

Das Gehäuse kann eine kompakte, sichere und wartungsfreundliche Unterbringung der wesentlichen Komponenten eines Gasaustauschsystems ermöglichen. Auf diese Weise kann eine tragbare, von einem Patienten mitführbare Gasaustauschvorrichtung bereitgestellt werden.

In vorteilhaften Weiterbildungen der Erfindung liegt das Verhältnis der Flussrate der ersten Pumpe zu der Flussrate der zweiten Pumpe im Bereich zwischen 4:1 und 6:1. Vorzugsweise beträgt das Flussratenverhältnis ca. 5:1. Durch ein derartiges Einstellen der Flussraten der jeweiligen Pumpen kann eine optimierte CO₂-Reduktionsrate erzielt bzw. eingestellt werden.

Ein Antrieb der ersten Pumpe ist derart in der Gasaustauschvorrichtung angeordnet bzw. positioniert, dass eine Abwärme des Antriebs der ersten Pumpe einen einlassseitigen Abschnitt des ersten Fluidkreislaufs erwärmt. In Weiterbildung der Erfindung ist ein Antrieb der zweiten Pumpe derart in der Gasaustauschvorrichtung angeordnet bzw. positioniert, dass eine Abwärme des Antriebs der zweiten Pumpe einen einlassseitigen Abschnitt des ersten Fluidkreislaufs erwärmt. Dazu kann die erste und ggf. die zweite Pumpe Befestigungsmittel aufweisen, die ein derartiges Anordnen in der Gasaustauschvorrichtung ermöglichen. Beispielsweise kann zumindest eine Zuflussleitung in einer örtlichen Nähe zu wenigstens einer ersten Pumpe, einer zweiten Pumpe oder auch einer ersten und einer zweiten Pumpe angeordnet werden. Eine Anordnung kann auch in unmittelbarer Nähe eines oder beider der Antriebe der ersten und zweiten Pumpe erfolgen. Es ist auch denkbar, dass eine Auslassleitung des durch den Gasaustauschprozess erwärmten Fluids entlang oder abschnittsweise um die Zuflussleitung des Fluids geführt ist. Auf diese Weise kann wiederum einer Erwärmung des angesaugten Fluids erfolgen. Durch die die Erwärmung der Zuflussleitung bzw. Gasleitung ergibt sich der Vorteil, dass sich kein Kondenswasser bilden kann, welches die Hohlfasern im Oxygenator als kleine Tröpfchen teilweise verschließen könnte. Ein teilweises Verschließen des Gasaustauschers bzw. Oxygenators kann die Effektivität des Gasaustausches dramatisch verringern, weshalb bekannte Oxgenatoren daher mehrmals am Tag entlüftet werden sollten.

Vorzugsweise ist eine Einrichtung zur aktiven Temperatursteuerung vorgesehen, die wenigstens einen in dem Gehäuse angeordneten Temperatursensor, wenigstens einen Lüfter sowie eine Regeleinrichtung aufweist. Mittels der Einrichtung zur aktiven Temperatursteuerung kann die Innentemperatur im Gehäuse im Betriebszustand z.B. in einem Temperaturbereich von 34° und 42° C, vorzugsweise in einem Temperaturbereich von 35° und 38° C eingeregelt werden. Die Abwärme der Pumpe bzw. der Pumpen wird dabei über die Luftströmung im Gehäuse zur Erwärmung der Komponenten verteilt und ein Überschuss an Wärme kann über die Gehäuseöffnung an die Umgebung abgegeben werden. Hierdurch kann auch auf einfache Weise eine Temperierung des angesaugten Fluids erzielt werden. Die Regeleinrichtung ist dabei vorzugsweise in die Steuereinrichtung der Gasaustauschvorrichtung integriert, wobei die Regeleinrichtung den Lüfter in Bezug auf Strömungsgeschwindigkeit und Strömungsrichtung anhand der Messdaten des wenigstens einen Temperatursensors steuert.

Von Vorteil ist es ferner, wenn der Lüfter zwischen einer Pumpe und einer Gehäuseöffnung, vorzugsweise in kurzer Distanz (kleiner 3 cm) sowohl zur Pumpe als auch zur Gehäuseöffnung, angeordnet ist, wodurch eine schnelle Wärmeabfuhr aus dem Gehäuse ermöglicht wird.

Durch eine derartige Anordnung der Pumpen kann Energie eingespart werden, wenn eine Erwärmung des ersten Fluids vor einem Gasaustausch gewünscht ist. Dies kann die Batterielaufzeit verlängern. Zudem kann eine Gasaustauschrate mit einem erwärmten Fluid während des Gasaustauschs verbessert sein.

Auslassseitig, insbesondere an einem Auslassabschnitt, des ersten Fluidkreislaufs kann ein Schalldämpfer zum Dämpfen einer Geräuschentwicklung aufgrund des Ausstoßes des ersten Fluids vorgesehen sein. Dadurch kann ein Geräuschpegel des Pumpsystems reduziert werden. Dies kann eine Lärmbelästigung, insbesondere bei dem mobilen Einsatz des Pumpsystems reduzieren und den Nutzungskomfort erhöhen.

In einigen Weiterbildungen umfasst das Pumpsystem wenigstens entweder einen Drucksensor und/oder einen Gasflusssensor und/oder einen Luftblasensensor. Dies kann eine Steuerung des Pumpsystems und eine Fehlererkennung verbessern. Dadurch kann die Zuverlässigkeit verbessert und das Einsatzgebiet des Pumpsystems erhöht werden. So kann das Fluid zuverlässig ausschließlich zur Abreicherung einer Komponente der Flüssigkeit eingesetzt werden, ohne dass gleichzeitig eine Anreicherung mit einer anderen Komponente erfolgen soll.

Die erste Pumpe des Pumpsystems kann vorteilhafterweise als eine Saugpumpe ausgebildet sein. Dies kann es einerseits erlauben, kleinere, leichtere und leisere Pumpenkomponenten mit entsprechend kleinerem Energieverbrauch für das Pumpsystem zu verwenden. Zudem kann mittels einer Saugpumpe zuverlässig verhindert werden, dass aufgrund des Saugprinzips Bestandteile, insbesondere Gasbestandteile, des Fluids in die Flüssigkeit übertreten.

Die tragbare Gasaustauschvorrichtung kann eine autarke Fluidversorgung zur Versorgung der Gasaustauschvorrichtung mit dem Fluid aufweisen. Dabei kann von der autarken Fluidversorgung insbesondere Umgebungsluft zur Verfügung gestellt werden. Die Verwendung von Umgebungsluft kann insbesondere deshalb von Vorteil sein, da ein Kohlenstoffdioxidgehalt in der Umgebungsluft weitaus geringer ist als ein Kohlenstoffdioxidgehalt in beispielsweise venösem Blut. Es kann somit lediglich durch Strömen von Umgebungsluft durch den ersten Fluidkreislauf eine Kohlenstoffdioxid-Abreicherung in Blut, das durch den zweiten Fluidkreislauf strömt, bewirkt werden. Entsprechend müssen, zur Abreicherung des Blutes von Kohlenstoffdioxid, keine schweren Kompressionsflaschen zur Gasversorgung der Gasaustauschvorrichtung von einem Patienten mitgeführt werden, wenn eine ECCO₂R-Anwendung indiziert ist. Es ist in diesem Fall ausreichend, Umgebungsluft anzusaugen und durch den ersten Fluidkreislauf zu strömen. Dies kann eine Tragbarkeit und Mobilität für einen Patienten weiter erhöhen.

"Autark" ist in diesem Zusammenhang so zu verstehen, dass eine externe Zuleitung von Gas, beispielsweise durch mitgeführte Gasflaschen, nicht notwendig ist. Eine Versorgung mit Gas erfolgt vielmehr unabhängig von einer mitgeführten Gasversorgung. Es versteht sich, dass in einigen Ausführungsformen der Erfindung dennoch eine Gasversorgung, beispielsweise einer Sauerstoffdruckflasche, vorzugsweise eine Sauerstoffdruckflasche geringer Größe, an die Gasaustauschvorrichtung anschließbar ist. Es ist zudem denkbar, dass ein Mischbetrieb aus einer autarken Gasversorgung und einer mitgeführten Gasversorgung durchführbar ist. Eine Steuerung des Betriebs bzw. des Mischbetriebs kann dabei auch abhängig von Sensorergebnissen oder Blutgasmessungen, vorzugsweise automatisch, erfolgen. Eine Kombination dieser Gasversorgungen kann auch zeitlich begrenzt vorgesehen sein.

Der erste bzw. der zweite Gasaustauschabschnitt der Gasaustauschvorrichtung können dabei im Wesentlichen mit dem ersten bzw. dem zweiten Fluidkreislauf des Pumpsystems zusammenfallen. Insofern kann das Pumpsystem mit dem ersten und zweiten Fluidkreislauf als eigenständige Komponente vorgesehen sein. Alternativ können der erste und zweite Fluidkreislauf fester Bestandteil der Gasaustauschvorrichtung sein und somit Bestandteile des ersten bzw. des zweiten Gasaustauschabschnitts bilden. Zudem ist es auch denkbar, dass der erste und zweite Fluidkreislauf unmittelbar in das Gehäuse der Gasaustauschvorrichtung integriert und untrennbar damit verbunden sind. Dies kann auch für lediglich einen der Fluidkreisläufe zutreffen, beispielsweise für den ersten Fluidkreislauf.

Die erfindungsgemäße tragbare Gasaustauschvorrichtung, insbesondere mit einer autarken Fluidversorgung, erlaubt einen mobilen Einsatz einer Gasaustauschvorrichtung. Dies kann erhebliche Vorteile für Anwender haben, beispielsweise für Patienten, die auf einen extrakorporalen Gasaustausch des Blutes angewiesen sind. Vorteilhafterweise kann der extrakorporale Gasaustausch mit der erfindungsgemäßen Vorrichtung auch dauerhaft erfolgen.

Die tragbare Gasaustauschvorrichtung weist in einigen Weiterbildungen eine Steuereinheit auf, die ausgebildet ist, die Flussrate wenigstens einer Pumpe der Pumpeinheit, also der ersten und/oder zweiten Pumpe des Pumpsystems, zu steuern. Dabei kann die Steuereinheit für die erste und/oder zweite Pumpe der Gasaustauschvorrichtung vorteilhafterweise integral mit einer Steuereinheit für die gesamte Gasaustauschvorrichtung ausgebildet sein. Dies kann eine flexible Anpassung der Pumpleistungen erlauben und somit auch eine Anpassung an individuelle Verhältnisse oder Bedürfnisse eines Nutzers der Gasaustauschvorrichtung.

In einigen Weiterbildungen der Erfindung ist die Wandung der tragbaren Gasaustauschvorrichtung zwischen dem ersten Gasaustauschabschnitt und dem zweiten Gasaustauschabschnitt so ausgebildet, dass diese zusätzlich für wenigstens ein zweites Gas passierbar ist. In alternativen Ausführungsformen können die Gasaustauschvorrichtung und/oder das Pumpsystem auch einen dritten Gasaustauschabschnitt bzw. einen dritten Fluidkreislauf aufweisen, worin ein weiteres Fluid strömen kann. Dieses weitere Fluid kann beispielsweise Sauerstoff sein oder, im Vergleich zu Umgebungsluft, einen erhöhten Sauerstoffgehalt enthalten. Es ist auch denkbar, dass das weitere Fluid komplexere Bestandteile aufweist, wie beispielsweise biochemisch-reaktive Stoffe wie Medikamente oder ähnliches. Insbesondere kann auch die Verabreicherung beispielsweise von Narkosegasen während einer Operation mittels einer erfindungsgemäßen Vorrichtung ermöglicht werden.

Die tragbare Gasaustauschvorrichtung kann wenigstens ein Überwachungsmittel mit wenigstens entweder einem Drucksensor und/oder einem Blutflussmesser und/oder einem Luftblasensensor umfassen. Das wenigstens eine Überwachungsmittel kann dabei insbesondere auch mit der Steuereinheit verbunden sein. Dabei weist die Steuereinheit vorzugsweise wenigstens einen Indikator zum Anzeigen eines Messwertes und/oder zum Darstellen einer Fehlfunktion der Gasaustauschvorrichtung auf. Vorzugsweise umfasst die Steuereinheit eine Anzeigeeinheit zum Darstellen von Informationen und/oder Fehlfunktionen sowie diverser Betriebsparameter. Das Überwachungsmittel der Gasaustauschvorrichtung kann dabei auch integral mit dem Überwachungsmittel eines in der Gasaustauschvorrichtung vorgesehenen Pumpsystems ausgebildet sein. Das Überwachungsmittel kann eine verbesserte Überprüfung der Funktionsfähigkeit der Gasaustauschvorrichtung bzw. einzelner Komponenten davon erlauben. Insbesondere kann das Vorsehen eines Überwachungsmittels erlauben, einen Regelkreis in der Gasaustauschvorrichtung oder auch in dem Pumpsystem bereitzustellen. Somit kann eine zuverlässige Versorgung mit Fluid in der Gasaustauschvorrichtung sichergestellt werden.

Der Blutflussmesser bzw. Luftblasensensor erlaubt die Erfassung des Blutflusses durch die Gasaustauschvorrichtung. Auf diese Weise kann eine Funktion der Gasaustauschvorrichtung überwacht werden. Der Luftblasensensor erfasst Luftblasen, die in der Flüssigkeit, insbesondere in dem Blut, vorliegen. Dies kann wiederum die Sicherheit der Vorrichtung erhöhen, beispielsweise indem verhindert wird, dass Luftblasen aus dem extrakorporalen Bereich in einen Blutkreislauf in dem Körper transportiert werden. In spezifischen Ausführungen kann auch nur ein Blutflusssensor oder nur ein Luftblasensensor oder jeweils ein Blutflusssensor und ein Luftblasensensor separat voneinander vorgesehen sein.

In Weiterbildungen der Erfindung kann zu dem ersten und zweiten, gegebenenfalls auch zu dem dritten, Fluidkreislauf, jeweils ein redundanter Fluidkreislauf vorgesehen sein. So kann bei Ausfall eines dieser Kreisläufe, insbesondere in einem Fall, in dem sofortige medizinische Maßnahmen an einem auf die Gasaustauschvorrichtung angewiesenen Patienten nicht durchgeführt werden können, das redundante System die Funktion des Gasaustausches alleine übernehmen. Eine Steuer- bzw. Regeltechnik zur Anpassung der Flussraten der jeweiligen Pumpen kann dazu beispielsweise automatisch die entsprechenden Flussraten des redundanten Systems erhöhen.

Auf dieselbe Weise kann eine Erhöhung der Flussraten zumindest in begrenztem Maße einer Effizienzreduzierung des Gasaustauschers entgegenwirken. Eine solche Effizienzreduzierung kann beispielsweise die Folge sein, wenn Blut durch einen Gasaustauscher geleitet wird und Blutbestandteile beginnen, an der Wandung zwischen dem ersten und dem zweiten Fluidkreislauf anzuhaften und dadurch einen Gastransport durch die Wandung verhindern.

Im Übrigen ist es möglich, wiederum zumindest in begrenztem Maße, eine, beispielsweise automatische, Reduzierung einer oder beider Flussraten der Fluide zu bewirken, beispielsweise auf ein medizinisch-notwendiges Minimum. Dies kann notwendig sein, falls die Leistung der Energieversorgung zu gering wird und eine Möglichkeit der Wiederaufladung nicht unmittelbar gegeben ist.

Es versteht sich, dass in Ausführungsformen, in der die Gasaustauschvorrichtung das erfindungsgemäße Pumpsystem aufweist, die Energieversorgung in Form von einer oder mehrerer, vorzugsweise aufladbarer, Batterien das Pumpsystem, die Steuereinheit sowie weitere mit Strom zu versorgende Komponenten gemeinsam speist.

Die Steuereinheit der tragbaren Gasaustauschvorrichtung kann über Strom- und/oder Datenkabel mit der Gasaustauschvorrichtung verbunden sein. Dabei kann eine Energieversorgung der Steuereinheit und der Gasaustauschvorrichtung in der Steuereinheit vorgesehen sein. Es ist auch denkbar, dass die Stromversorgung in dem Gehäuse der Gasaustauschvorrichtung vorgesehen ist, oder dass in dem Gehäuse der Gasaustauschvorrichtung zumindest ein Teil einer Stromversorgung vorgesehen ist. In einem solchen Fall ist es auch vorstellbar, dass die Steuereinheit der Gasaustauschvorrichtung kabellos mit der Gasaustauschvorrichtung verbunden ist. Dazu kann die Steuereinheit sowie die Gasaustauschvorrichtung eine Sende- und/oder Empfangseinheit aufweisen. Dies kann den Komfort bei der Bedienung der Gasaustauschvorrichtung erhöhen.

Zudem ist es denkbar, dass die Steuereinheit selbst ein modulares System darstellt, das zumindest eine Feststation und eine tragbare Steuereinheit umfasst. Die Feststation und die tragbare Steuereinheit können in diesem Fall wiederum kabellos miteinander verbunden sein. Auf diese Weise können Daten, beispielsweise aktuelle Pumpraten, Temperaturen der Fluide und/oder Vitalparameter und ähnliches, soweit diese Daten erfasst werden, von der tragbaren Steuereinheit an die Feststation übermittelt werden. Zudem kann die Feststation beispielsweise Steuerbefehle zur Steuerung der Gasaustauschvorrichtung an die tragbare Steuereinheit übermitteln.

Auf diese Weise kann ein Bewegungsradius eines Patienten erhöht werden. Für eine lokale Datenverbindung zwischen der tragbaren Steuereinheit und der Feststation kann beispielsweise ein Bewegungsradius von 50 m oder mehr relativ zu der Feststation ermöglicht werden.

An der Steuereinheit kann des Weiteren ein Mittel vorgesehen sein, das eine Befestigung der Steuereinheit an der tragbaren Gasaustauschvorrichtung erlaubt. An der Gasaustauschvorrichtung kann dazu ein komplementäres Mittel vorgesehen werden, so dass beispielsweise ein Befestigungsabschnitt der Steuereinheit mit einem Befestigungsabschnitt der Gasaustauschvorrichtung in Eingriff geraten und eine zuverlässige Befestigung der Gasaustauschvorrichtung ermöglichen kann. Die Befestigung der Steuereinheit kann beispielsweise an dem Gehäuse der Gasaustauschvorrichtung vorgesehen sein. Alternativ kann eine Befestigung der Steuereinheit auch an einem Tragesystem der Gasaustauschvorrichtung vorgesehen sein. Zudem kann eine Befestigung der Steuereinheit auch unmittelbar an der Kleidung eines Patienten erfolgen. Zudem kann das Befestigungsmittel der Steuereinheit auch dazu vorgesehen sein, die Steuereinheit an einer Tragestruktur, beispielsweise an einem Patientenbett, oder einem Tragesystem für medizinische Geräte in einem Krankenzimmer zu befestigen.

Insbesondere kann das Befestigungsmittel der Steuereinheit eine hakenförmige Ausbildung haben, die ein Einhaken des Befestigungsmittels an einem komplementären Aufnahmeabschnitt oder beispielsweise an einem rohrförmigen Element oder einer Außenkontur des Gehäuses der Gasaustauschvorrichtung ermöglicht. Zudem ist eine Befestigung an einer flachen Struktur, wie beispielsweise einer Rollstuhllehne oder ähnlichem denkbar. Dazu kann das Befestigungsmittel der Steuereinheit mit einer Federvorspannung ausgebildet sein, so dass eine Klemmung aufgrund der Federvorspannung des Befestigungsmittels eine ausreichende Haltekraft zur Befestigung der Steuereinheit bereitstellt. Im Übrigen kann das Befestigungsmittel oder ein zusätzliches Standmittel derart ausgebildet und an der Steuereinheit ausgebildet sein, dass die Steuereinheit auf einer Unterlage, beispielsweise einem Tisch oder einem Rollcontainer oder ähnlichem, aufgestellt werden kann.

Soweit eine Feststation und eine tragbare Steuereinheit vorgesehen sind, kann die Feststation einen Anschlussabschnitt aufweisen, der zur Aufnahme oder zumindest zur Verbindung der tragbaren Steuereinheit vorgesehen ist. Auf diese Weise kann die tragbare Steuereinheit, beispielsweise wenn sich ein Patient in der Nähe der Feststation aufhält, mit der Feststation verbunden werden. Die Feststation kann dazu insbesondere eine Ladevorrichtung zum Aufladen der in der tragbaren Steuereinheit vorgesehenen Batterien aufweisen. So kann, beispielsweise während einer Ruhephase des Patienten, wie insbesondere in der Nacht, die tragbare Steuereinheit aufgeladen werden, ohne dass ein Betrieb der Gasaustauschvorrichtung unterbrochen werden muss.

### Kurze Beschreibung der Zeichnungen

Die Erfindung sowie vorteilhafte Weiterbildungen davon werden nun anhand bestimmter in den beigefügten Zeichnungen dargestellter Ausführungsbeispiele erläutert, in denen gleiche Merkmale mit den gleichen Bezugszeichen versehen sind. Es zeigt:
- Figur 1: eine tragbare Gasaustauschvorrichtung gemäß einer Ausführungsform der Erfindung mit einem Pumpsystem;
- Figur 2: ein Beispiel einer Steuereinheit gemäß einer Ausführungsform der Erfindung.

### Beschreibung der Ausführungsbeispiele der Erfindung

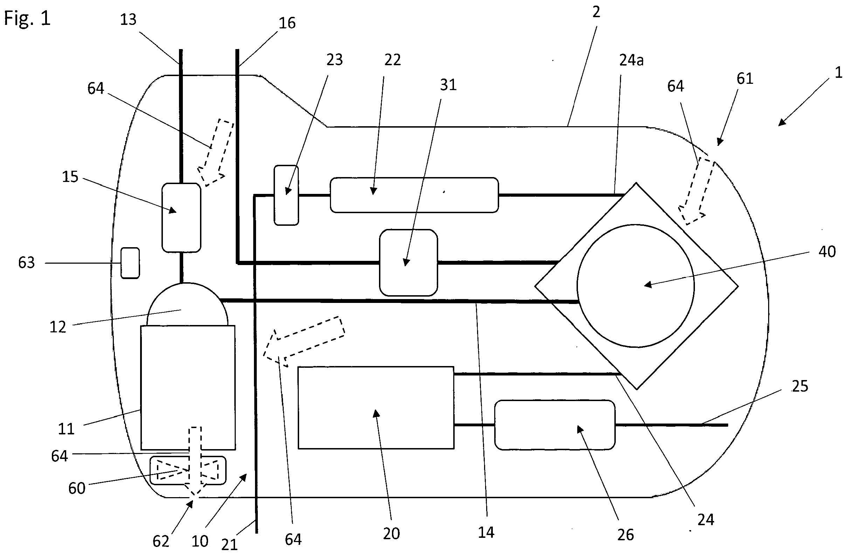

Figur 1 zeigt eine tragbare Gasaustauschvorrichtung 1 gemäß einer Ausführungsform der Erfindung. Die Gasaustauschvorrichtung 1 dient dem Gasaustausch zwischen einem Fluid und einer Flüssigkeit als zweitem Fluid. Um einen Gasaustausch zwischen den Fluiden zu ermöglichen, ist in der Gasaustauschvorrichtung 1 ein Pumpsystem 10 vorgesehen.

Das Pumpsystem 10 weist eine erste Pumpe 20 auf, die in der in Figur 1 dargestellten Ausführungsform als eine Gaspumpe ausgebildet ist. Eine zweite in Figur 1 dargestellte Pumpe 11 ist vorliegend eine Flüssigkeitspumpe, die zum Pumpen einer biologischen Flüssigkeit, insbesondere Blut, ausgelegt ist. Die erste Pumpe 20 weist dabei ein Leistungsgewicht kleiner 30 kg/KW bei einem Pumpengewicht von höchstens 0,5 kg, vorzugsweise höchstens 0,3 kg auf, während die zweite Pumpe 11 ein Leistungsgewicht kleiner 25 kg/KW bei einem Pumpengewicht von höchstens 0,7 kg, vorzugsweise höchstens 0,5 kg aufweist.

Die zweite Pumpe 11 wird im Folgenden auch als eine Blutpumpe bezeichnet. Die Pumpfunktion der zweiten Pumpe 11 übernimmt dabei in der Regel eine Zentrifugalpumpe, die aus einem Pumpenantrieb und einem über eine Magnetkopplung berührungslos angetriebenen Pumpenkopf 12 besteht. Weiter geeignete Pumpen wären Kreiselpumpen, wie Halbaxialpumpen (auch als Diagonalpumpen bezeichnet). Der Pumpenkopf 12, der im Folgenden auch als Blutpumpenkopf bezeichnet sein kann, ist mit einer Zuflussleitung 13 verbunden. Durch die Zuflussleitung 13 gelangt Flüssigkeit, beispielsweise Blut von Seiten eines Patienten, in die Gasaustauschvorrichtung 1. Im stromabwärtigen Verlauf der Blutpumpe 11 wird die Flüssigkeit als zweites Fluid, vorliegend also das Blut, über eine zweite Leitung 14 in einen Gasaustauscher 40, dargestellt als eine Mischkammer, geleitet. Nach dem Durchtreten durch den Gasaustauscher 40 wird die Flüssigkeit über eine Ableitung 16 zurück zu einer Patientenseite geleitet. In der Zuflussleitung 13 ist zudem ein so genannter In-Line Drucksensor 15 ausgebildet. Der Drucksensor 15 erlaubt die Bestimmung eines Drucks der Flüssigkeit, hier von Blut, in der Zuflussleitung 13 und somit, zumindest indirekt, auch eine Flussrate hin zu der zweiten Pumpe 11. Zudem erlaubt der Drucksensor 15 eine Bestimmung des so genannten Drainagedrucks, also des Druckes zwischen dem Gefäßzugang und dem Pumpenkopf 12 der Blutpumpe 11. Es kann mehr als ein Drucksensor 15 vorgesehen sein, so z.B. drei Drucksensoren 15. Ein Drucksensor vor der Pumpe 11, ein Drucksensor zwischen der Pumpe 11 und dem Gasaustauscher 40 und ein Drucksensor in Flussrichtung hinter dem gasauistauscher 40. Die Zuflussleitung 13, die Blutpumpe 11, die zweite Leitung 14 sowie die Ableitung 16 sind Teil eines zweiten Gasaustauschabschnitts. Die Gesamtlänge der Leitungen des zweiten Fluids bzw. Bluts beträgt vorzugsweise weniger als 100 cm, noch bevorzugter zwischen 50 und 80 cm. Der Leitungs- bzw. Schlauchweg außerhalb des Gehäuses 2 von und zum Patienten beträgt zwischen 0,9 und 1,7 m, vorzugsweise zwischen 1 und 1,6 m.

Die erste Pumpe 20 ist als eine Gaspumpe ausgebildet. Die erste Pumpe 20 weist dazu in der in Figur 1 gezeigten Ausführungsform zwei Gaszuflussschläuche 21 auf. Die Gaszuflussschläuche 21 dienen der Versorgung des Pumpsystems 10 und der Gasaustauschvorrichtung 1 insgesamt mit dem Gas, das in dem Gasaustauscher 40 mit der von der zweiten Pumpe 11 geförderten Flüssigkeit in Wechselwirkung gerät. Die Gaszuflussschläuche 21 bilden somit einen Teil eines ersten Gasaustauschabschnitts der Gasaustauschvorrichtung 1. Die Gaszuflussleitungen 21 sind in der Gasaustauschvorrichtung 1 räumlich derart angeordnet, dass sie, hier zumindest abschnittsweise, in räumlicher Nähe zu der zweiten Pumpe 11 verlaufen. Dadurch kann die Abwärme der zweiten Pumpe 11 das in den Gaszuflussleitungen 21 strömende Gas erwärmen.

Das in dieser Ausführungsform verwendete Gas in dem ersten Gasaustauschabschnitt kann in besonderen Ausführungsformen auch eine Flüssigkeit sein, so dass hier allgemein auch von einem Fluid gesprochen werden kann. In der gezeigten Ausführungsform ist das Gas insbesondere Umgebungsluft, also eine bekannte Zusammensetzung verschiedener Gase der Atemluft, die insbesondere einen nur geringen Anteil an CO₂ aufweist.

Die erste Pumpe 20 fördert das Gas durch eine Gasaustauscherleitung 24, 24a zu dem Gasaustauschabschnitt 40 der Gasaustauschvorrichtung 1, der die Mischkammer bildet. Dabei wird das Gas in der gezeigten Ausführungsform zunächst durch einen Gasflusssensor 22 geleitet. Dies kann damit auch eine Bestimmung der Menge des einströmenden Gases und dessen Flussrate erlauben. Nach Durchtritt durch den Gasflusssensor 22 wird das Gas durch einen Partikelfilter 23 geleitet. Der Partikelfilter 23 ist derart ausgelegt, dass feste Bestandteile, wie Staub, Pollen, oder weitere Schwebeteilchen oder Verschmutzungen aus dem Gasstrom ausgefiltert werden. Der Partikelfilter 23 kann auch an einer anderen Position des gebildeten ersten Gasaustauschabschnitts angeordnet sein, beispielsweise bereits stromaufwärts von dem Gasflusssensor 22, insbesondere auch stromaufwärts von der ersten Pumpe 20. Es ist auch denkbar, dass eine Mehrzahl von Filtern an verschiedenen Positionen des ersten Gasaustauschabschnitts ausgebildet sind. Bei einer Mehrzahl von Filtern kann ein Filtersystem vorgesehen werden, das sukzessive Partikel verschiedener Größen ausfiltert. So kann erreicht werden, dass eine Wahrscheinlichkeit des Verstopfens des ersten Gasaustauschabschnitts zumindest reduziert wird.

Das Gas, das durch den Gasaustauscher 40 geströmt ist, strömt erneut durch die erste Pumpe 20. Die erste Pumpe 20 ist dazu in bevorzugten Ausführungsformen als eine Saugpumpe ausgebildet. Dabei saugt die erste Pumpe 20 den Gasstrom durch den gesamten ersten Gasaustauschabschnitt bis zu der Auslassleitung 25. Während eine derartige Saugpumpe den Gasstrom mittels eines Unterdrucks durch den Gasaustauscher 40 befördert, ist es in alternativen Ausführungsformen auch denkbar, dass die erste Pumpe 20 eine Überdruckpumpe ist. Dabei würde der Gasstrom entsprechend mittels eines Überdrucks durch den Gasaustauscher bzw. die Mischkammer 40 befördert werden.

Die Auslassleitung 25 ist dabei der ersten Pumpe 20 nachgeschaltet. Zwischen einem in der Figur 1 nicht erkennbaren Auslass und der ersten Pumpe 20 ist zudem ein Schalldämpfer 26 vorgesehen. Der Schalldämpfer 26 ist dazu ausgelegt, den Lärmpegel des austretenden Gasstromes aus dem Auslass zu dämpfen und somit eine Geräuschentwicklung der Gasaustauschvorrichtung 1 zu reduzieren.

In dem zweiten Fluidkreislauf bzw. in dem zweiten Gasaustauschabschnitt ist in der in Figur 1 gezeigten Ausführungsform des Weiteren ein Blutfluss- und Luftblasensensor 31 ausgebildet.

Der eigentliche Gasaustausch in dem Gasaustauscher 40 zwischen der Flüssigkeit, hier vorzugsweise Blut, und dem Fluid, hier vorzugsweise Umgebungsluft, erfolgt, wie es aus dem Stand der Technik bekannt ist. Gemäß einer speziellen Ausführung der vorliegenden Erfindung soll mit der erfindungsgemäßen Vorrichtung lediglich eine Decarboxylierung ohne gleichzeitige Oxygenierung vorgenommen werden. Erfindungsgemäß sind die Komponenten des Pumpsystems 10 sowie die zugehörigen Komponenten wie Gasaustauscher 40, Zu- und Ableitungen 13, 14, 16; 21, 24, 24a, 25 und weitere Komponenten auf Volumenströme im Bereich unter 2 l/min, bevorzugt im Bereich von ca. 0,3-1 l/min, ausgelegt. Zudem sind die Komponenten derart dimensioniert, dass extrakorporale Volumina, beispielsweise extrakorporales Blutvolumen, gering gehalten wird. Um bei den erfindungsgemäß geringen Volumenströmen eine suffiziente Durchströmung und einen effektiven Gasaustausch zu gewährleisten, wird in dem Gasaustauscher 40 beispielsweise ein Faserbündel mit einer Vielzahl an individuellen Hohlfasern vorgesehen. Verwendbare Faserbündel, die an die Gaszu- bzw. -abflüsse sowie an die Blutzu- bzw. -abflüsse angeschlossen werden können, sind aus dem Stand der Technik bereits bekannt und werden an dieser Stelle nicht beschrieben.

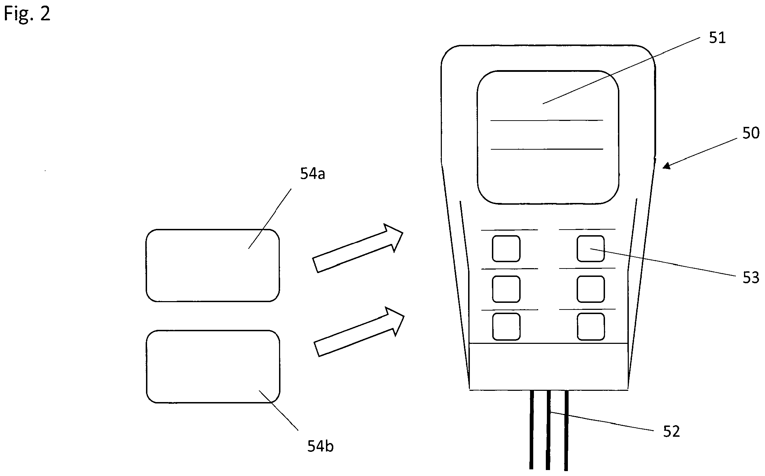

Der Gasflusssensor 22 kann mit einer Steuereinrichtung 50 der Gasaustauschvorrichtung 1 verbunden sein, die in Figur 2 beispielhaft dargestellt ist. Der Gasflusssensor kann mit der zweiten Pumpe 20 und/oder mit der ersten Pumpe 11 verbunden sein. Der Drucksensor 15 und/oder die zweite Pumpe 11 können mit der Steuereinheit 50 verbunden sein. Auf diese Weise kann ein Kontrollkreis zur Regulierung der Gasflussrate und/oder der Blutflussrate, bzw. allgemein der Fluidflussrate und/oder der Flüssigkeitsflussrate, geschaffen werden. Es versteht sich, dass es auch denkbar ist, lediglich einen Steuerschaltkreis, ohne einen Regelkreis, zu schaffen.

Die Steuereinrichtung 50 weist in der gezeigten Ausführungsform eine Anzeigeeinheit 51 auf. Es ist in dieser gezeigten Ausführungsform vorgesehen, dass die Anzeigeeinheit 51 verschiedene Informationen wie Blutflussrate, Gasflussrate bzw. Pumpleistung der in der Gasaustauschvorrichtung 1 enthaltenen Pumpen 11, 20 darstellt. An der Steuereinheit 50 ist eine Mehrzahl an Leitungen 52 vorgesehen. Die Leitungen 52 beinhalten dabei Datenleitungen von und zu den verschiedenen Bauteilen wie Sensoren und Pumpen in der Gasaustauschvorrichtung. Zudem kann wenigstens eine der Leitungen 52 zur Energieversorgung der Steuereinheit 50 dienen.

Des Weiteren weist die Steuereinheit 50 eine Mehrzahl an Bedientasten 53 auf. Die Bedientasten erlauben beispielsweise die Auswahl und das Verändern verschiedener Parameter, die zur Steuerung oder Regelung der Gasaustauschvorrichtung 1 relevant sind.

In der gezeigten Ausführungsform sind in der Steuereinheit 50 zwei wiederaufladbare Batterien 54a, 54b vorgesehen. Diese Batterien 54a, 54b und die Steuereinheit 50 sind dabei derart ausgebildet, dass eine Stromversorgung der Steuereinheit auch ausschließlich über eine der Batterien erfolgen kann. Auf diese Weise ist eine örtliche Nähe zu einer externen Stromversorgung nicht zu jedem Zeitpunkt notwendig. Im Übrigen erlaubt diese Ausbildung, dass eine der Batterien 54b aus der Steuereinheit 50 entfernt und beispielsweise wieder aufgeladen werden kann, während die andere Batterie 54a in der Steuereinheit 50 verbleibt und diese sowie die entsprechenden Komponenten der Gasaustauschvorrichtung 1 mit Energie versorgt. Zudem kann eine Aufladung der Batterien auch in einem in die Steuereinheit eingesetzten Zustand durch die Energieversorgung der Steuereinheit erfolgen.

Das Gehäuse 2 der Gasaustauschvorrichtung 1 weist ein maximales Innenraumvolumen von bevorzugt 9500 cm³ auf, bei einer bevorzugten maximalen durchschnittlichen Tiefe (Erstreckung des Gehäuses 2 in Richtung senkrecht zur Figuren-Ebene in Fig. 1) von 15 cm und bei einem bevorzugten durchschnittlichen summarischen Wert für Höhe und Breite von zusammengenommen maximal 65 cm. Die durchschnittliche Höhe oder die durchschnittliche Breite betragen hierbei bevorzugt maximal 40 cm. Die Tiefe beträgt dabei bevorzugt 1/4 bis 1/8 des summarischen Wertes der durchschnittlichen Höhe und der durchschnittlichen Breite zusammengenommen, so dass das Gehäuse 2 einen gedrungenen bis flächigen Habitus verwirklicht.

Die Gasaustauschvorrichtung weist ferner eine Einrichtung zur aktiven Temperatursteuerung auf. Diese umfasst wenigstens einen in dem Gehäuse 2 angeordneten Temperatursensor 63, wenigstens einen Lüfter 60 sowie eine Regeleinrichtung, die in die Steuereinrichtung 50 integriert ist. Der Lüfter 60 ist in dem Gehäuse 2 zwischen einer Gehäuseöffnung 62 und der zweiten Pumpe 11 angeordnet. Der Lüfter 60 ist vorzugsweise in geringem Abstand von <3 cm sowohl zur Pumpe als wie auch zur Gehäuseöffnung im Gehäuse 2 angeordnet. Über den Lüfter 60 kann im Gehäuse 2 ein Luftstrom erzeugt werden, dessen Zirkulation zwischen den zwei oder mehr vorgesehenen Gehäuseöffnungen 61 und 62 durch die Pfeile 64 angedeutet ist. Mittels der Einrichtung zur aktiven Temperatursteuerung kann die Innentemperatur im Gehäuse 2 im Betriebszustand in einem Temperaturbereich von 34° und 42° C, vorzugsweise in einem Temperaturbereich von 35° und 38° C eingeregelt werden. Die Abwärme der Pumpen 11, 20 wird dabei über die Luftströmung im Gehäuse zur Erwärmung der Komponenten verteilt und ein Überschuss an Wärme über die Gehäuseöffnung 62 an die Umgebung abgegeben. Der Lüfter kann dabei in unterschiedlichen Strömungsrichtungen betrieben werden, um entweder mehr Wärme im Gehäuse zu verteilen oder um Wärme schneller aus dem Gehäuse abzuführen. In Figur 1 ist über die Pfeile 64 die Strömungsrichtung zum schnellen Wärmeabtransport aus dem Gehäuse angedeutet. Die gegensätzliche Strömungs- bzw. Lüftungsrichtung würde eine stärkere Wärmeverteilung im Gehäuse bewirken. Muss der zu behandelnde Patient "gekühlt" werden, so ist auch eine niedrigere Zieltemperatur als 35° C möglich.

Das Gehäuse 2 ist zur Aufnahme der Komponenten des ersten Gasaustauschabschnitts und des zweiten Gasaustauschabschnitts vorgesehen und vorbereitet. Dazu kann das Gehäuse 2 eine Vielzahl von Ausnehmungen in einem Gehäuseeinsatz aufweisen. Die Ausnehmungen sind derart dimensioniert, dass die entsprechenden Komponenten der Gasaustauschabschnitte formschlüssig in den Ausnehmungen in dem Gehäuseeinsatz aufgenommen werden können. Auf diese Weise kann eine sichere Aufnahme der Komponenten der Gasaustauschabschnitte erfolgen, so dass insbesondere Relativbewegungen, Verschleiß und das Lösen von Verbindungen wirkungsvoll reduziert werden kann.

Das Gehäuse 2 kann einen Gehäuseeinsatz aufweisen, der Aufnahmeabschnitte insbesondere für die erste und zweite Pumpe 20, 11, die Mischkammer 40 sowie die Zu- und Ableitungen der Fluidkreisläufe und weiteres aufweist. Bereits auf diese Weise kann in dieser gezeigten Ausführungsform eine Schall- sowie eine Temperaturisolierung erfolgen. Der Gehäuseeinsatz ist derart dimensioniert, dass dieser in das Gehäuse passgenau einsetzbar ist.

### Bezugszeichenliste

- 1: Gasaustauschvorrichtung
- 2: Gehäuse
- 2a: Gehäuseausnehmungen
- 2b: Gehäuseeinsatz
- 10: Pumpsystem
- 11: zweite Pumpe
- 12: Pumpenkopf
- 13: Zuleitung der Flüssigkeit
- 14: zweite Leitung
- 15: In-Line Drucksensor
- 16: Ableitung der Flüssigkeit
- 20: erste Pumpe
- 21: Gaszuflussschlauch / Zuflussleitung des Fluids
- 22: Gasflusssensor
- 23: Partikelfilter
- 24/24a: Gasaustauscherleitung
- 25: Auslassleitung des Fluids
- 26: Schalldämpfer
- 31: Blutfluss- und Luftblasensensor
- 40: Gasaustauscher/Mischkammer
- 50: Steuereinrichtung
- 52: Leitungen
- 53: Bedientasten
- 54a/54b: wiederaufladbare Batterien
- 60: Lüfter
- 61: Gehäuseöffnung
- 62: Gehäuseöffnung
- 63: Temperatursensor
- 64: Pfeile (Luftstrom)

## Patentansprüche

1. Tragbare Gasaustauschvorrichtung (1) zum Gasaustausch wenigstens eines Gases aus einer Flüssigkeit mit einem Fluid, aufweisend:
- einen ersten Gasaustauschabschnitt (21, 24, 24a, 26) zum Durchfluss eines Fluids,
- einen zweiten Gasaustauschabschnitt (13, 14, 16) zum Durchfluss einer mit einem ersten Gas angereicherten Flüssigkeit, wobei das Fluid eine geringere Konzentration des Gases aufweist als die Flüssigkeit,
- ein Pumpsystem (10), das zumindest zum Transport des Fluids mit einer geringen Konzentration des ersten Gases in dem ersten Gasaustauschabschnitt in einem ersten Fluidkreislauf, ausgebildet ist, wobei das Pumpsystem (10) eine erste Pumpe (20) zum Pumpen des Fluids aufweist,
- eine Mischkammer (40), in der der erste Gasaustauschabschnitt (21, 24, 24a, 26) und der zweite Gasaustauschabschnitt (13, 14, 16) durch eine zumindest von dem ersten Gas passierbar ausgebildete Wandung getrennt aneinander angrenzen,
- ein Gehäuse (2) zur Aufnahme wenigstens des Pumpsystems (10) und der Mischkammer (40), und
- eine Heizvorrichtung zum Aufwärmen des Fluids,
**dadurch gekennzeichnet, dass**
die Heizvorrichtung dadurch bereitgestellt ist, dass ein Antrieb der ersten Pumpe (20) derart in dem Gehäuse (2) der Gasaustauschvorrichtung (1) positionierbar ist, dass eine Abwärme des Antriebs der ersten Pumpe (20) einen einlassseitigen Abschnitt des ersten Fluidkreislaufs erwärmt, wobei der einlassseitige Abschnitt in stromaufwärtiger Richtung des ersten Fluidkreislaufs vor dem ersten Gasaustauschabschnitt ausgebildet ist.

2. Tragbare Gasaustauschvorrichtung (1) nach Anspruch 1, wobei der erste Gasaustauschabschnitt (21, 24, 24a, 26) einen Partikelfilter (23) umfasst und wobei das Fluid im Wesentlichen Umgebungsluft, Sauerstoff oder eine Mischung aus Umgebungsluft und Sauerstoff aufweist.

3. Tragbare Gasaustauschvorrichtung (1) nach Anspruch 2, wobei der erste Gasaustauschabschnitt (21, 24, 24a, 26) einlassseitig einen Partikelfilter (23) umfasst.

4. Tragbare Gasaustauschvorrichtung (1) nach einem der vorhergehenden Ansprüche, wobei das Pumpsystem (10) eine zweite Pumpe (11) zum Pumpen einer Flüssigkeit durch den zweiten Gasaustauschabschnitt, vorzugsweise durch einen zweiten Fluidkreislauf, aufweist.

5. Tragbare Gasaustauschvorrichtung (1) nach Anspruch 1, wobei die Heizvorrichtung zum Warmhalten der Flüssigkeit ausgebildet ist.

6. Tragbare Gasaustauschvorrichtung (1) nach Anspruch 4, wobei ein Antrieb der zweiten Pumpe (20) derart in dem Gehäuse (2) der Gasaustauschvorrichtung (1) positionierbar ist, dass eine Abwärme des Antriebs der zweiten Pumpe einen einlassseitigen Abschnitt des ersten Fluidkreislaufs erwärmt.

7. Tragbare Gasaustauschvorrichtung (1) nach einem der vorhergehenden Ansprüche, wobei in dem Pumpsystem (10) die erste Pumpe (20) und/oder die zweite Pumpe (11) ein Steuermittel (50) zum Steuern der Flussrate des Fluids aufweist.

8. Tragbare Gasaustauschvorrichtung (1) nach Anspruch 7, wobei die Flussrate der ersten Pumpe (20) zwischen ca. 1-12 l/min oder 1 - 8 l/min einstellbar ist.

9. Tragbare Gasaustauschvorrichtung (1) nach einem der Ansprüche 7 oder 8, wobei die Flussrate der zweiten Pumpe (11) geringer als 2 l/min oder zwischen ca. 0,2-1,5 l/min einstellbar ist.

10. Tragbare Gasaustauschvorrichtung (1) nach Anspruch 9, wobei eine maximale Förderrate der zweiten Pumpe (11) weniger als 4 l/min oder weniger als 2 l/min oder höchstens 1,5 l/min beträgt.

11. Tragbare Gasaustauschvorrichtung (1) nach einem der Ansprüche 1 bis 8, wobei die erste Pumpe (20) eine Pumpe mit einem Leistungsgewicht kleiner 30 kg/KW bei einem maximalen Pumpengewicht von 0,5 kg ist.

12. Tragbare Gasaustauschvorrichtung (1) nach einem der Ansprüche 4, 7, 9 und 10, wobei die zweite Pumpe (11) eine Pumpe mit einem Leistungsgewicht kleiner 25 kg/KW bei einem maximalen Pumpengewicht von 0,7 kg ist.

13. Tragbare Gasaustauschvorrichtung (1) nach einem der Ansprüche 4 und 6 bis 10, wobei das Verhältnis der Flussraten der ersten Pumpe (20) zu der zweiten Pumpe (11) im Bereich zwischen 4:1 und 6:1 liegt oder ca. 5:1 beträgt.

14. Tragbare Gasaustauschvorrichtung (1) nach einem der Ansprüche 1 bis 13, wobei eine Einrichtung zur aktiven Temperatursteuerung vorgesehen ist, die wenigstens einen in dem Gehäuse (2) angeordneten Temperatursensor (63), wenigstens einen Lüfter (60) sowie eine Regeleinrichtung aufweist.

15. Tragbare Gasaustauschvorrichtung (1) nach Anspruch 14, wobei der Lüfter (60) zwischen einer Pumpe (11, 20) und einer Gehäuseöffnung (62) vorgesehen ist.

16. Tragbare Gasaustauschvorrichtung (1) nach einem der vorhergehenden Ansprüche, wobei auslassseitig des ersten Fluidkreislaufs ein Schalldämpfer (26) zum Dämpfen einer Geräuschentwicklung vorgesehen ist.

17. Tragbare Gasaustauschvorrichtung (1) nach Anspruch 16, wobei auslassseitig des ersten Fluidkreislaufs an einem Auslassabschnitt der Schalldämpfer (26) zum Dämpfen einer Geräuschentwicklung vorgesehen ist.

18. Tragbare Gasaustauschvorrichtung (1) nach einem der vorhergehenden Ansprüche, wobei die Gasaustauschvorrichtung (1) des Weiteren wenigstens entweder einen Drucksensor (15) und/oder einen Gasflusssensor (22) und/oder einen Luftblasensensor (31) umfasst.

19. Tragbare Gasaustauschvorrichtung (1) nach einem der vorhergehenden Ansprüche, wobei die erste Pumpe (20) eine Saugpumpe ist und/oder wobei die Gasaustauschvorrichtung (1) eine autarke Fluidversorgung zur Versorgung der Gasaustauschvorrichtung (1) mit dem Fluid umfasst.

20. Tragbare Gasaustauschvorrichtung (1) nach einem der vorhergehenden Ansprüche, die des Weiteren eine Steuereinheit (50) umfasst, die ausgebildet ist, die Flussrate wenigstens einer Pumpe (11, 20) der Pumpeinrichtung (10) zu steuern.

21. Tragbare Gasaustauschvorrichtung (1) nach einem der vorhergehenden Ansprüche, wobei die Wandung zwischen dem ersten Gasaustauschabschnitt (21, 24, 24a, 26) und dem zweiten Gasaustauschabschnitt (13, 14, 16) passierbar für wenigstens ein zweites Gas ausgebildet ist.

## Claims

1. Portable gas exchange device (1) for gas exchange of at least one gas from a biological liquid with a fluid, having
- a first gas exchange section (21, 24, 24a, 26) for passage of the fluid,
- a second gas exchange section (13, 14, 16) for passage of the biological liquid enriched with a first gas, wherein the fluid has a lower concentration of the gas than the liquid,
- a pumping system (20) that is designed at least for the transport of the fluid with a low concentration of the first gas in the first gas exchange section in a first fluid circuit, wherein the pump system (10) has a first pump (20) for pumping the fluid,
- a mixing chamber (40) in which the first gas exchange section (21, 24, 24a, 26) and the second gas exchange section (13, 14, 16) adjoin one another, separated by a wall, which is permeable at least to the first gas,
- a housing (2) as a receptacle for at least the pumping system (10) and the mixing chamber (40), and
- a heating device for warming the fluid,
**characterized in that**
the heating device is provided such that a drive of the first pump (20) can be positioned in the housing (2) of the gas exchange device (1), such that a waste heat of the drive of the first pump (20) warms an inlet-side section of the first fluid circuit, wherein the inlet-side section is formed in the upstream direction of the first fluid circuit prior to the first gas exchange section.

2. Portable gas exchange device (1) according to claim 1, wherein the first gas exchange section (21, 24, 24a, 26) comprises a particle filter (23), and
wherein the fluid essentially has ambient air, oxygen, or a mixture of ambient air and oxygen.

3. Portable gas exchange device (1), wherein the first gas exchange section (21, 24, 24a, 26) comprises at the inlet side a particle filter (23).

4. Portable gas exchange device (1) according to one of the preceding claims, wherein the pumping system (10) has a second pump (11) for pumping a liquid through the second gas exchange section, preferably through a second fluid circuit.

5. Portable gas exchange device (1) according to claim 1, wherein the heating device is configured for keeping the liquid warm.

6. Portable gas exchange device (1) according to claim 4, wherein the drive of the second pump (11) can be positioned in the housing (2) of the gas exchange device (1) such that the waste heat of the drive of the second pump warms an inlet-side section of the first fluid circuit.

7. Portable gas exchange device (1) according to one of the preceding claims, wherein, in the pumping system (10), the first pump (20) and/or the second pump (11) has a control means (50) for controlling the flow rate of the fluid.

8. Portable gas exchange device (1) according to claim 7, wherein the flow rate of the first pump (20) is adjustable to between approx. 1-12 l/min or 1-8 l/min.

9. Portable gas exchange device (1) according to claim 7 or 8, wherein the flow rate of the second pump (11) is less than 2 l/min or is adjustable between about 0.2-1.5 l/min.

10. Portable gas exchange device (1) according to claim 9, wherein the maximum supply rate of the second pump (11) is less than 4 I/min or less than 2 l/min or no more than 1.5 l/min.

11. Portable gas exchange device (1) according to one of claims 1 to 8, wherein the first pump (20) is a pump having a weight-to-power ratio of less than 30 kg/KW at a maximum pump weight 0.5 kg.

12. Portable gas exchange device (1) according to one of claims 4, 7, 9 and 10, wherein the second pump (11) is a pump having a weight-to-power ratio of less than 25 kg/KW at a maximum pump weight of 0.7 kg.

13. Portable gas exchange device (1) according to one of claims 4 and 6 to 10, wherein the ratio of the flow rates of the first pump (20) to the second pump (11) is in a range of between 4:1 and 6:1 or is approx. 5:1.

14. Portable gas exchange device (1) according to one of claims 1 through 13, wherein a device for active temperature control is provided that has at least one temperature sensor (63) arranged in the housing (2), at least one ventilating fan (60), and a governor control device.

15. Portable gas exchange device (1) according to claim 14, wherein the ventilating fan (60) is arranged between a pump (11, 20) and a housing opening (62).

16. Portable gas exchange device (1) according to one of the preceding claims, wherein a noise suppressor (26) for dampening a noise emission is provided at the outlet side of the first fluid circuit.

17. Portable gas exchange device (1) according to claim 16, wherein the noise suppressor (26) is provided at the outlet section of the first fluid circuit at an outlet section for dampening noise emission.

18. Portable gas exchange device (1) according to one of the preceding claims, wherein the gas exchange device (1) further comprises at least either a pressure sensor (15) and/or a gas flow sensor (22) and/or an air bubble sensor (31).

19. Portable gas exchange device (1) according to one of the preceding claims, wherein the first pump (20) is a suction pump and/or the gas exchange device (1) comprises an autarkic fluid supply for supplying the gas exchange device (1) with the fluid.

20. Portable gas exchange device (1) according to one of the preceding claims, which further comprises a control unit (50) that is designed to control the flow rate of at least one pump (11, 20) of the pumping device (10).

21. Portable gas exchange device (1) according to one of the preceding claims, wherein the wall between the first gas exchange section (21, 24, 24a, 26) and the second gas exchange section (13, 14, 16) is designed so as to be permeable to at least one second gas.

## Revendications

1. Dispositif d'échange de gaz portatif (1) pour l'échange d'au moins un gaz à partir d'un liquide avec un fluide, comprenant :
- une première portion d'échange de gaz (21, 24, 24a, 26) pour le passage d'un fluide,
- une deuxième portion d'échange de gaz (13, 14, 16) pour le passage d'un liquide enrichi avec un premier gaz, dans lequel le fluide présente une concentration en gaz inférieure à celle du liquide,
- un système de pompage (10) qui est conçu au moins pour le transport du fluide avec une faible concentration en premier gaz dans la première portion d'échange de gaz dans un premier circuit de fluide, dans lequel le système de pompage (10) comprend une première pompe (20) pour le pompage du fluide,
- une chambre de mélange (40), dans laquelle la première portion d'échange de gaz (21, 24, 24a, 26) et la deuxième portion d'échange de gaz (13, 14, 16) sont adjacentes entre elles et sont séparées d'une paroi conçue de façon à pouvoir être traversée au moins par le premier gaz,
- un boîtier (2) pour le logement d'au moins le système de pompage (10) et la chambre de mélange (40) et
- un dispositif de chauffage pour le chauffage du fluide,
**caractérisé en ce que**
le dispositif de chauffage est mis à disposition grâce au fait qu'un entraînement de la première pompe (20) peut être positionné dans le boîtier (2) du dispositif d'échange de gaz (1) de façon à ce que la chaleur dégagée par l'entraînement de la première pompe (20) chauffe une portion côté entrée dans la direction amont du premier circuit de fluide avant la première portion d'échange de gaz.

2. Dispositif d'échange de gaz portatif (1) selon la revendication 1, dans lequel la première portion d'échange de gaz (21, 24, 24a, 26) comprend un filtre à particules (23) et dans lequel le fluide comprend globalement de l'air ambiant, de l'oxygène ou un mélange d'air ambiant et d'oxygène.

3. Dispositif d'échange de gaz portatif (1) selon la revendication 2, dans lequel la première portion d'échange de gaz (21, 24, 24a, 26) comprend un filtre à particules (23) du côté de l'entrée.

4. Dispositif d'échange de gaz portatif (1) selon l'une des revendications précédentes, dans lequel le système de pompage (10) comprend une deuxième pompe (11) pour le pompage d'un liquide à travers la deuxième portion d'échange de gaz, de préférence à travers un deuxième circuit de fluide.

5. Dispositif d'échange de gaz portatif (1) selon la revendication 1, dans lequel le dispositif de chauffage est conçu pour maintenir le liquide au chaud.

6. Dispositif d'échange de gaz portatif (1) selon la revendication 4, dans lequel un entraînement de la deuxième pompe (20) peut être positionné dans le boîtier (2) du dispositif d'échange de gaz (1) de façon à ce qu'une chaleur dégagée par l'entraînement de la deuxième pompe chauffe une portion côté entrée du premier circuit de fluide.

7. Dispositif d'échange de gaz portatif (1) selon l'une des revendications précédentes, dans lequel, dans le système de pompage (10), la première pompe (20) et/ou la deuxième pompe (11) comprend un moyen de commande (50) pour le contrôle du débit du fluide.

8. Dispositif d'échange de gaz portatif (1) selon la revendication 7, dans lequel le débit de la première pompe (20) est réglable entre environ 1-12 l/min ou 1 - 8 l/min.

9. Dispositif d'échange de gaz portatif (1) selon l'une des revendications 7 ou 8, dans lequel le débit de la deuxième pompe (11) est réglable à moins de 2 l/min ou entre environ 0,2 - 1,5 l/min.

10. Dispositif d'échange de gaz portatif (1) selon la revendication 9, dans lequel un débit de la deuxième pompe (11) est inférieur à 4 l/min ou inférieur à 2 l/min ou est de 1,5 l/min maximum.

11. Dispositif d'échange de gaz portatif (1) selon l'une des revendications 1 à 8, dans lequel la première pompe (20) est une pompe avec un rapport poids/puissance inférieur à 30 kg/kW, avec un poids de pompe maximum de 0,5 kg.

12. Dispositif d'échange de gaz portatif (1) selon l'une des revendications 4, 7, 9 et 10, dans lequel la deuxième pompe (11) est une pompe avec un rapport poids/puissance inférieur à 25 kg/kW, avec un poids de pompe maximum de 0,7 kg.

13. Dispositif d'échange de gaz portatif (1) selon l'une des revendications 4 et 6 à 10, dans lequel le rapport entre les débits de la première pompe (20) et de la deuxième pompe (11) se situe entre 4:1 et 6:1 ou est d'environ 5:1.

14. Dispositif d'échange de gaz portatif (1) selon l'une des revendications 1 à 13, dans lequel un dispositif pour le contrôle actif de la température est prévu, qui comprend au moins un capteur de température (63) disposé dans le boîtier (2), au moins un ventilateur (60) ainsi qu'un dispositif de régulation.

15. Dispositif d'échange de gaz portatif (1) selon la revendication 14, dans lequel le ventilateur (60) est prévu entre une pompe (11, 20) et une ouverture du boîtier (62).

16. Dispositif d'échange de gaz portatif (1) selon l'une des revendications précédentes, dans lequel, du côté de la sortie du premier circuit de fluide, est prévu un silencieux (26) pour l'atténuation des nuisances sonores.

17. Dispositif d'échange de gaz portatif (1) selon la revendication 16, dans lequel le silencieux (26) pour l'atténuation des nuisances sonores est prévu du côté de la sortie du premier circuit de fluide, au niveau d'une portion de sortie.

18. Dispositif d'échange de gaz portatif (1) selon l'une des revendications précédentes, dans lequel le dispositif d'échange de gaz (1) comprend en outre au moins soit un capteur de pression (15) et/ou un capteur de débit (22) et/ou un détecteur de bulles d'air (31).

19. Dispositif d'échange de gaz portatif (1) selon l'une des revendications précédentes, dans lequel la première pompe (20) est une pompe d'aspiration et/ou dans lequel le dispositif d'échange de gaz (1) comprend une alimentation en fluide autonome pour l'alimentation du dispositif d'échange de gaz (1) en fluide.

20. Dispositif d'échange de gaz portatif (1) selon l'une des revendications précédentes, qui comprend en outre une unité de commande (50) qui est conçue pour contrôler le débit d'au moins une pompe (11, 20) du dispositif de pompage (10).

21. Dispositif d'échange de gaz portatif (1) selon l'une des revendications précédentes, dans lequel la paroi entre la première portion d'échange de gaz (21, 24, 24a, 26) et la deuxième portion d'échange de gaz (13, 14, 16) est conçu de façon à pouvoir être traversée par au moins un deuxième gaz.
